Europäisches Patentamt

European Patent Office

Office européen des brevets

(11) Publication number: 0 310 436
A2

# EUROPEAN PATENT APPLICATION

(21) Application number: 88309136.5

(22) Date of filing: 30.09.88

(51) Int. Cl.⁴: C 12 N 15/00
C 12 R 1/465

(30) Priority: 30.09.87 GB 8723000

(43) Date of publication of application:
05.04.89 Bulletin 89/14

(84) Designated Contracting States:
AT BE CH DE FR GB GR IT LI LU NL SE

(71) Applicant: Antibioticos, S.A.
Bravo Murillo 38
28015 Madrid (ES)

(72) Inventor: Vega, Diego Pulido Centro De Biol. Molecular
Universidad Autonoma De Madrid Cantoblanco
E-28049 Madrid (ES)

Martinez, Antonio Gimenez Centro De Biol.Molecular
Universidad Autonoma De Madrid Cantoblanco
E-28049 Madrid (ES)

Falgueras, Margarita S. Centro De Biol. Molecular
Universidad Autonoma De Madrid Cantoblanco
E-28049 Madrid (ES)

Mellado, Rafael Perez Centro De Biol. Molecular
Universidad Autonoma De Madrid Cantoblanco
E-28049 Madrid (ES)

Redondo, Asuncion Martin Centro De Biol. Molecular
Universidad Autonoma De Madrid Cantoblanco
E-28049 Madrid (ES)

(74) Representative: Ablewhite, Alan James et al
MARKS & CLERK 57/60 Lincoln's Inn Fields
London WC2A 3LS (GB)

(54) Streptomyces expression vectors.

(57) The present invention relates to Streptomyces expression vectors containing heterologous terminator sequences, especially when derived from B. subtilis phage φ29, and to cells and expression systems using such vectors.

EP 0 310 436 A2

## Description

### STREPTOMYCES EXPRESSION VECTORS

The present invention relates to novel Streptomyces expression vectors having heterologous terminator sequences.

The gram-positive streptomycetes undergo a cycle of complex morphological differentiation. The nature of the regulatory signals involved in the mechanism of differentiation in streptomycetes is still not completely understood. Although their genomes have a high G + C content (about 73%), streptomycetes have an RNA polymerase that can recognise and use Escherichia coli promotors despite the fact that Streptomyces promoter sequences often show no homology with the -35 and -10 consensus sequences of the E. coli. The sequences of several Streptomyces promotors are now known.

In the gram-positive, spore-forming bacterium B. subtilis, the template specificity of RNA polymerase changes during differentiation, because of the production of a series of RNA polymerase sigma factors. As Streptomyces coelicolor contains more than one RNA polymerase holoenzyme, the regulation of differentiation in S. coelicolor may resemble that in Bacillus spp. S. coelicolor RNA polymerase recognizes, in vitro, a S. lividans promoter with -35 and -10 sequences that differ from those of E. coli. In addition, two B. subtilis promoters, one with E. coli -35 and -10 consensus sequences, the other without, are recognised by the different S. coelicolor RNA polymerases. Thus, the RNA polymerase holoenzymes present in streptomycetes may have analogues, not only among different species of the same genus, but also among different genera.

A DNA fragment from the B. subtilis phage φ29, containing the main early and late viral promoters, cloned in a S. lividans promoter-probe plasmid, is capable of being utilized to initiate transcription in vivo at the same sites as does B. subtilis RNA polymerase. These viral promoters show high activity in S. lividans.

However, terminator sequences of streptomycetes, both characterised and putative, are distinctive in having large stem-loop structures with low free energies (-30 to -80 kcal/mol). and no uridine-rich tails, as expected for a genome having a high G + C content.

The region 3' to the Streptomyces genes aph, tsr and vph, hyg and sph and at contains one or more IRS (inverted repeat sequences) which have been proposed as transcription termination signals, and the IRS's of the aph gene have been shown to have transcription termination activity in Streptomyces lividans. In addition, a 205 bp DNA fragment, containing an IR from the Streptomyces plasmid plJ101, acts as an in vivo terminator both in S. lividans and E. coli. Neither of these two terminators nor the putative terminator IRS of other Streptomyces genes is followed by a thymidine rich sequence, a particular feature of most of the Rho-independent terminators in E. coli.

Therefore, very little is yet known on how transcription is terminated in streptomycetes. The ampC terminator from the E. coli chromosome and the major terminator from E. coli phage fd are active terminators in vivo in Streptomyces, although in these cases little else is known about how this process occurs.

We have now established that a DNA fragment from the B. subtilis phage φ29, containing the bidirectional transcription terminator TD1, where the right early transcription and late viral transcription terminate, is efficiently recognized in Streptomyces spp. and terminates transcription at the same site as B. subtilis RNA polymerase.

Thus, in a first aspect of the present invention, there is provided a Streptomyces expression vector containing at least one heterologous, Rho-independent terminator. In particular, there is provided such a vector containing at least one Rho-independent terminator derived from B. subtilis phage φ29.

The term "expression vector" is used herein to describe any vector containing an expression sequence, or any vector adapted to contain such a sequence.

The terminator sequence derived from φ29 is preferably that sequence known as "TD1". Owing to the nature of the field, the invention also includes the use of the equivalent of such a sequence or a functional mutant form of either. By "equivalent" is meant a functional sequence similar to the original sequence but not necessarily derived therefrom, that is, the equivalent sequence may be partially or entirely synthetic. By "mutant" is meant a form of either the original or equivalent sequences which varies therefrom to any extent while still retaining the requisite termination characteristics. Such variation may be effected by, for example, insertion, inversion, deletion or mutagenesis.

Although it has been shown that S. lividans recognises φ29 promoters very efficiently, it is surprising that a φ29 terminator should be recognised, as it has an entirely different structure to that of naturally occurring Streptomyces terminators.

The ability of Streptomyces spp. to efficiently recognise heterologous Rho-independent terminators opens the way toward the use of streptomycetes in large-scale protein manufacture.

The use of the aforementioned terminators with heterologous promoters is preferred, especially when both are derived from B. subtilis phage φ29. It will be appreciated that promoters may also be the suitable equivalent or mutant forms.

Studies on phage φ29 transcription show the existence of a region (in the EcoRI-D fragment) where transcription initiated both at the right early promoters, C1 and C2, and at the main late promoter, A3, terminates. This terminator region is precisely located between nt 17265 and 17304 of the φ29 genome and acts in B. subtilis, infected with φ29, as a Rho-independent terminator signal in either orientation.

Streptomyces vectors containing at least one promoter and at least one terminator having a coding sequence therebetween are particularly useful for obtaining high levels of expression of encoded protein. Plasmids without a coding sequence but having at least one unique restriction site between promoter and terminator sequences would enable a coding sequence of choice to be inserted as required.

Plasmids exemplified herein contain the φ29 early promoters A2a, A2b and A2c and φ29 late promoter A3 in either orientation relative to the φ29 TD1 terminator. The parent plasmid is pIJ486 and there exist 3 unique endonuclease sites for XbaI, BamHI and Sst I between the two control sequences. These plasmids form particularly preferred embodiments of the present invention.

The following Examples serve to illustrate the invention and are not to be construed as limiting the invention in any way.

EXAMPLE 1

(a) Cloning of the φ29 Promoters in Plasmid pIJ486.

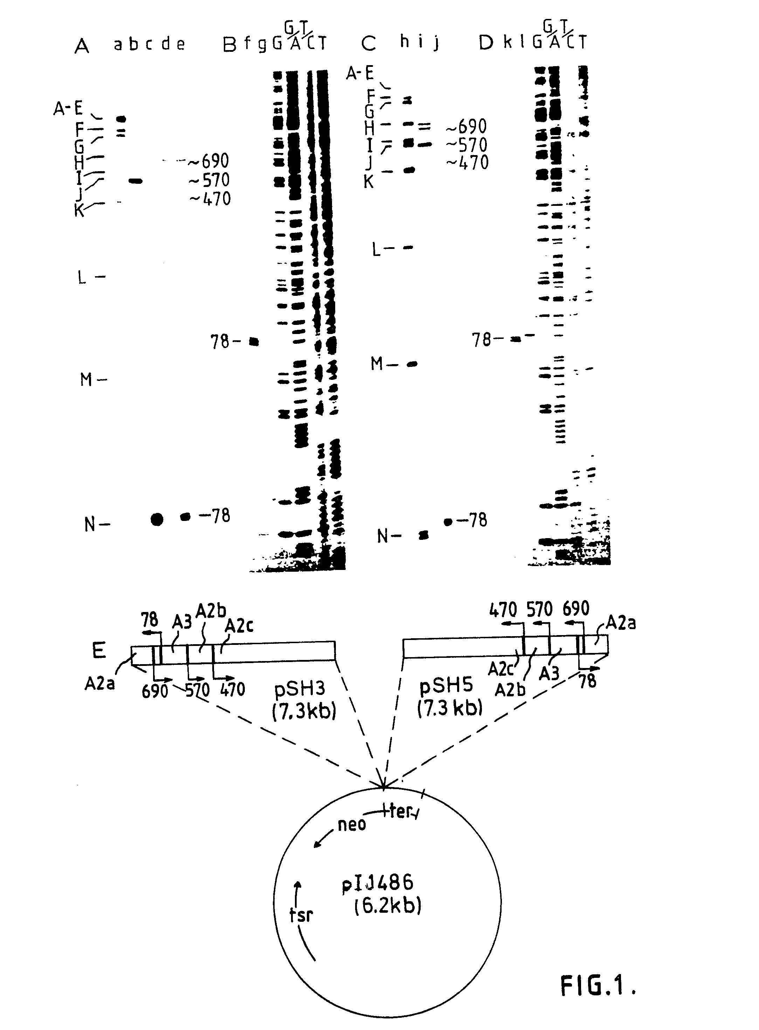

The 763-bp HindIII-H fragment of φ29 DNA, of known sequence, contains the early promoters A2a, A2b and A2c and the late promoter A3. To test the ability of RNA polymerase from streptomycetes to recognise heterologous promoters, the HindIII-H fragment of φ29 DNA was inserted into the Streptomyces promoter-probe plasmid pIJ486 at a unique HindIII site. This site is present in the linker sequences placed upstream of the Tn5-derived neo gene, carried by the plasmid. Since early transcription of φ29 DNA takes place from the light strand and late transcription from the heavy strand, the neo gene would be expected to be subject to control by the φ29 promoters in either orientation of the viral DNA fragment. Expression of the neo gene confers kanamycin resistance (Km^R). Plasmid pIJ486 also carries the gene for thiostrepton resistance (Tsr^R) as well as the major transcriptional terminator of the E. coli phage fd which prevents transcriptional read-through into the neo gene from vector promoters. The recombinant plasmids pSH3 and pSH5, having the neo gene under the control of the late and early φ29 promoters, respectively, are depicted in Fig. 1E. Both plasmids were shown to be stable upon propagation in selective media and no variations in their molecular sizes were detected (results not shown).

From previous studies of φ29 transcription, it was known where transcription initiates in vivo at the A2a, A2b, A2c and A3 promoters within the HindIII-H fragment. To check whether the S. lividans RNA polymerase would recognise the φ29 promoters in vivo, the viral HindIII-H fragment was purified, radioactively labelled at 5'-protruding ends with [γ-32P]ATP and the separated strands were hybridized with RNA extracted from B. subtilis infected with φ29 in the presence of chloramphenicol (Cm) (early strand) or in the absence of the drug (late strand). In the presence of Cm only early viral transcription can take place, since the later transcription depends on the synthesis of the product of the φ29 early gene 4,protein p4; in the absence of Cm, both early and late RNA can be made. RNA extracted from cultures of S. lividans 66 carrying the recombinant plasmids pSH3 or pSH5 was hybridized in the same way to the radioactively labelled strands of the φ29 HindIII-H fragment. After hybridization, the samples were subjected to S1 digestion and the protected oligodeoxynucleotides fractionated on denaturing PA gels (Figs. 1A and C). Transcripts initiating at the A2a, A2b, A2c and A3 φ29 promoters were found in S. lividans when the neo gene was controlled either by the φ29 early (Fig. 1C, i and j) or late (Figs. 1A, D and E) promoters. As is shown in Fig. 1A, B and C, transcription from the early strand in φ29-infected B. subtilis mainly takes place from the A2b promoter and, to a lesser extent, from the A2a and A2c promoters, and transcription from the late strand occurs from the late A3 promoter. The precise location of the φ29 transcription by B. subtilis or S. lividans RNA polymerase takes place at the same site, the oligodeoxynucleotides protected by RNA initiating at the A3 promoter were in all cases run on denaturing PA gels side by side with a set of products of sequencing reactions. Transcripts from the φ29 DNA coincided with those from the recombinant plasmids pSH3 (Fig. 1B, f and g) and pSH5 (Fig. 1D, k and l), clearly indicating that S. lividans RNA polymerase can specifically recognize the heterologous φ29 promoters.

(b) Expression Level of the neo Gene in the Recombinant Plasmids

To determine the efficiency of the φ29 promoters in S. lividans, the level of Km^R conferred by the expression of the neo gene was established. Under the control of the A3 promoter (plasmid pSH3), S. lividans formed colonies resistant to 150μg/ml of Km, whereas resistance was observed at 300μg/ml when the neo gene was under the control of the φ29 early promoters (plasmid pSH5). These Km^R levels could be considered high when compared with those conferred by similar multicopy plasmids carrying Streptomyces promoters. In agreement with these results, the APHII activity was higher in S. lividans harbouring the plasmid pSH5 than in the strain containing the plasmid pSH3 (Table 1). Thus, the amount of the neo gene product synthesized in S. lividans carrying the plasmid pSH5 accounts for nearly 6% of the total protein made, and 2% in cells carrying the plasmid pSH3. These levels were determined by densitometry of the stained PA gels after fractionation of total cellular proteins.

## TABLE 1

Expression of the neo gene encoded in the recombinant plasmids

| Cells | KmR (μg Km/ml)[a] | APHII activity(mU)[b] | APHII % total protein[c] |
|---|---|---|---|
| S. lividans 66 | <2 | 50 | - |
| S. lividans [plJ486] | <5 | 125 | - |
| S. lividans [pSH3] | 150 | 900 | 2 |
| S. lividans [pSH5] | 300 | 2200 | 6 |

[a] Determined by the capability of the cells to grow in Km-containing minimal agar plates (Hopwood, 1967)
[b] Determined as described elsewhere (Haas and Dowding, 1975); 1mU is equivalent to 1 pmol of phosphorylated Km per mg of protein per min.
[c] Determined by densitometry of the total cell proteins fractionated on an SDS-12% PA gel.

## EXAMPLE 2

### (a) Cloning of the φ29 TD1 Terminator in Plasmids pSH3 and pSH5

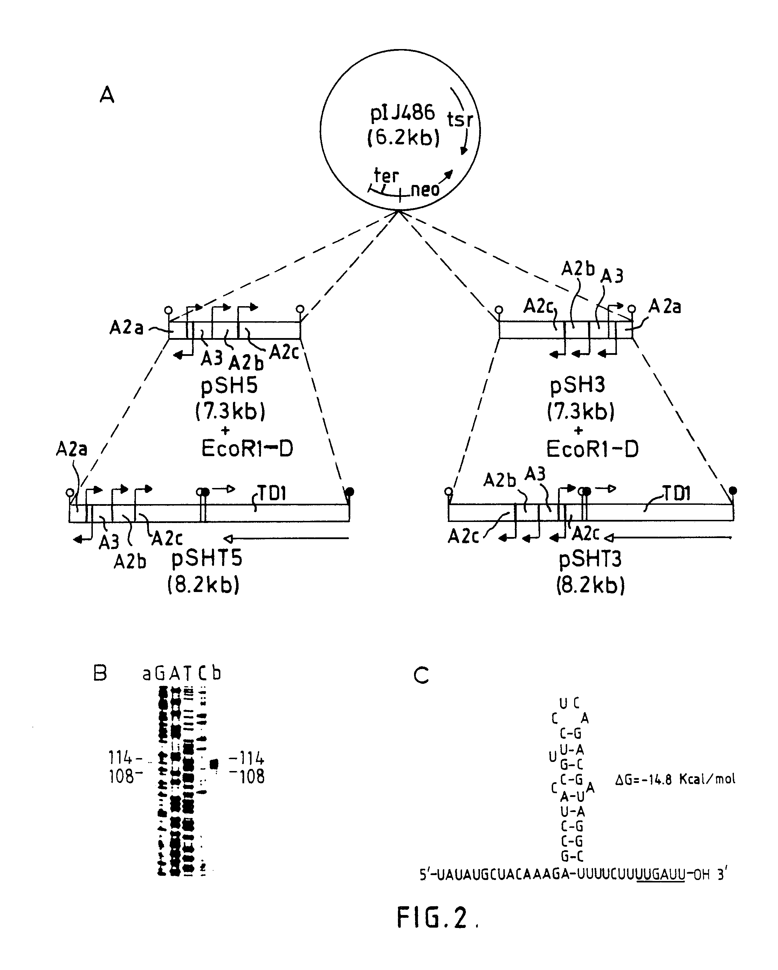

Plasmids pSH3 and pSH5 (Example 1) had the 9120bp EcoRI-D fragment of φ29 DNA, of known sequence (Garvey et al., 1985), containing the viral TD1 terminator, inserted in the unique EcoRI site (Fig. 2A). This site is contained in the linker sequences between the φ29 promoters and the Tn5-derived neo gene carried in the plasmids. Expression of the neo gene confers Km resistance to the cells harbouring the parental plasmids pSH3 or pSH5. Insertion of an effective terminator sequence should reduce, or abolish, this resistance.

The recombinant plasmids also carry the gene TsrR, as well as the major transcriptional terminator of the E. coli phage fd, which prevents transcriptional reading through into the neo gene from putative promoters present in the original plasmid PIJ486.

Out of the two possible orientations in which the φ29 DNA EcoRI-D fragment could be inserted, only one of them was obtained in each case, after screening 82 transformants. This was possibly due to the presence of an open reading frame coding for part of the viral protein p16 which, in the appropriate orientation, could be transcribed before the RNA polymerase reaches the viral terminator sequences; eventual synthesis of the p16 fragment could be deleterious to the host cell, making the insertion of the EcoRI-D fragment in this particular orientation not viable.

The recombinant plasmids pSH3 and pSH5, as well as their respective derivatives containing the φ29 TD1 terminator, pSHT3 and pSHT5, are shown in Fig. 2A. All these plasmids proved to be stable upon propagation in selective media and no variations in their molecular sizes were detected (results not shown).

From previous studies on φ29 transcription it was known that the right early transcription termination at the TD1 terminator occurred between nt 107 and 112 from the right end of the EcoRI-D fragment. To establish whether S. lividans also terminates at this site, the viral EcoRI-D fragment was purified, radioactively labelled at the 3'- recessed ends with [α-32P]dATP in the presence of PolIk and the separated strands were hybridized with RNA extracted from cultures of S. lividans 66 carrying the recombinant plasmids pSHT3 or pSHT5.

After hybridization, the samples were subjected to S1 digestion and the protected oligodeoxynucleotides fractionated on denaturing PA gels. Hybridization took place with one of the EcoRI-D fragment strands but not with the other, resulting in protection of the complete DNA strand (results not shown) and a set of oligodeoxynucleotides with sizes around 110 nt in both pSHT3 and pSHT5. This is the result expected if transcription termination takes place within the DNA fragment, with some of the transcripts running through the termination signal and reaching the end of the DNA fragment.

To locate this termination site precisely, the oligodeoxynucleotides protected by RNA initiating at the φ29 promoters were in both cases run on denaturing PA gels, side by side with a set of products of DNA sequencing reactions. Transcripts initiated at the viral promoters in S. lividans mainly terminate between nt 108-114 from the right end of the φ29 EcoRI-D fragment in both cases (Fig. 2B lanes a and b), coinciding with the position where transcription terminates in the TD1 terminator in B. subtilis cultures infected with φ29, indicating that the φ29 TD1 transcription termination signal is also recognised in vivo by S. lividans.

### (b) Expression Level of the neo Gene in Plasmids pSHT3 and pSHT5

To determine the efficiency of transcription termination of the TD1 sequences in S. lividans, the level of Km resistance conferred by the expression of the neo (KmR) gene was measured. Table II shows that, when the terminator sequences were present (plasmids pSHT3 and pSHT5), the Km resistance levels dropped about 7-fold relative to the levels of Km resistance conferred by the parental plasmids pSH3 and pSH5, respectively. In agreement with these results, the APHII activity was also severely reduced in cells carrying plasmids pSHT3 and pSH5 (Table II).

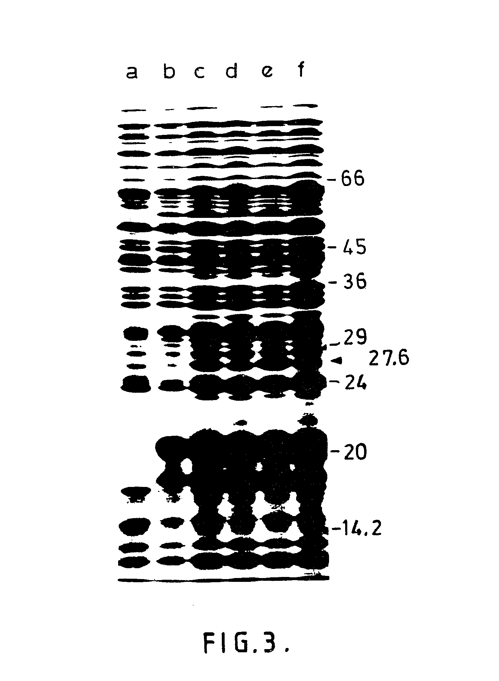

The neo gene product synthesized in S. lividans carrying plasmids pSH5 or pSH3 accounted for nearly 6% and 2.5% of the total protein in the ribosome-free supernatants, respectively, whereas these values dropped to 0.9% and 0.5% when S. lividans carried the plasmids pSHT5 and pSHT3, respectively (Table II). These levels were determined by densitometry of the stained PA gels after fractionation of total cellular protein (Fig. 3). The reduction in the levels of Km resistance conferred by plasmids pSHT3 or pSHT5 was not due to a decrease in the plasmids copy number which can also be inferred from the relative amounts of the plasmid-specific proteins shown in Fig. 3.

TABLE 2

Expression of the neo gene encoded in the recombinant plasmids

| Cells | $Km^R$ ($\mu g$ Km/ml)[a] | APHII activity(mU)[b] | APHII % total protein[c] |
|---|---|---|---|
| S. lividans 66 | <2 | 50 | - |
| S. lividans [plJ486] | <5 | 125 | - |
| S. lividans [pSH3] | 150 | 900 | 2.5 |
| S. lividans [pSHT3] | 20 | 160 | 0.5 |
| S. lividans [pSH5] | 300 | 2200 | 6.0 |
| S. lividans [pSHT5] | 45 | 410 | 0.9 |

[a] Determined by the capability of the cells to grow in Km-containing minimal agar plates (Hopwood, 1967)
[b] Determined as described elsewhere (Haas and Dowding, 1975); 1mU is equivalent to 1 pmol of phosphorylated Km per mg of protein per min.
[c] Determined by densitometry of the total cell proteins fractionated on an SDS-12% PA gel.

S. lividans strains [pSHT3] and [pSHT5] have been deposited with the Torry Research Station, Aberdeen, on 30 September, 1987, and carry deposit no's NCIB 12550 and NCIB 12551, respectively.

In Fig. 2, the vertical bars at the extensions represent the φ29 promoters. The direction of transcription is indicated by arrows; arrows with open arrowheads indicate transcription termination whereas arrows with blackened arrowheads indicate transcription initiation. ↑ EcoRI; ↑, HindIII. DNA fragments protected from S1 digestion by the RNA made in S. lividans harbouring plasmid pSHT3 (lane a) or pSHT5 (lane b), fractionated by electrophoresis on denaturing 6% PA-7 M urea gels. Sizes of the S1-protected fragment are given in nt. Sequencing reactions (Sanger et al., 1977) are from the right terminal AccI fragment of φ29 DNA of known sequence (Escarmis and Salas, 1981; Yoshikawa et al., 1981) and were used as size standards. The RNA from stationary-phase cultures of S. lividans was prepared as described by Jaurin and Cohen (1984).

Fig. 3 shows the synthesis of the neo gene product encoded in the recombinant plasmids. The proteins made in S. lividans harbouring the vector plJ486 (lane b) or the recombinant plasmids pSH3 (lane c), pSHT3 (lane d), pSH5 (lane e), pSHT5 (lane f), or carrying no plasmid (lane a), were fractionated by electrophoresis on an 0.1% SDS-12% PA gel from the corresponding S100 extracts (Skinner and Cundliffe, 1980). Numbers at the right margin are molecular weight markers given in kD. Markers from top of bottom are, bovine albumin, egg albumin, glyceraldehyde 3-phosphate dehydrogenase, carbonic anhydrase, trypsinogen, soybean trypsin inhibitor and α-lactalbumin. The inferred molecular size for the APHII protein (27.6 kD) agrees with the one previously reported (Beck et al., 1982).

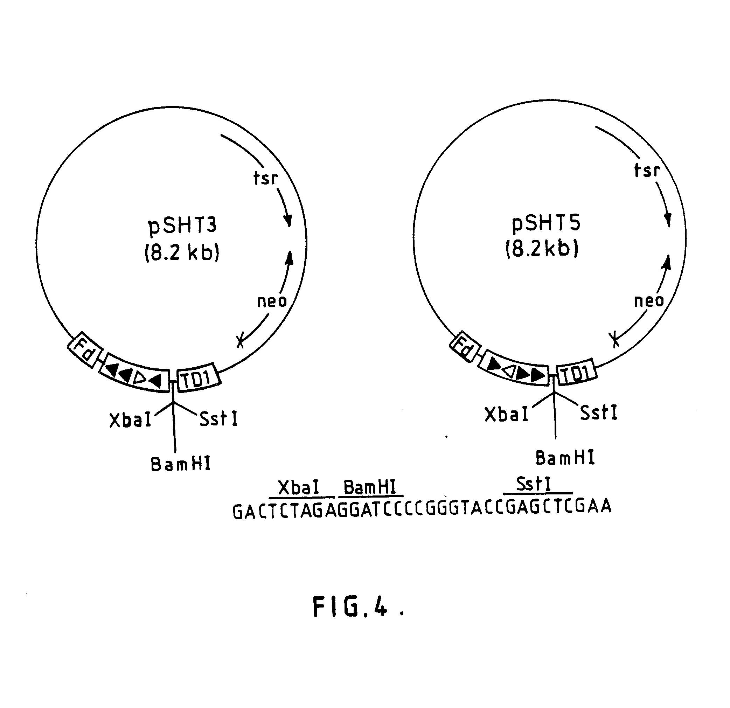

Fig. 4 is a diagrammatic representation of plasmids pSHT3 and pSHT5 showing the orientation and placement of certain features of the plasmids. Fd indicates the location of the phange fd major transcriptional terminator, TD1 the position of the φ29 major transcriptional terminator, ▶ φ29 early promoters (A2a, A2b, A2c) and ▷ the φ29 late promoter (A3).

**Claims**

1. A Streptomyces expression vector containing at least one heterologous, Rho-independent terminator sequence.

2. A vector according to claim 1 wherein he terminator sequence is derived from B. subtilis phage φ29, or is equivalent thereto or is a functional mutant form of either.

3. A vector according to claim 1 or 2 wherein the termination sequence corresponds to the TD1 sequence of φ29.

4. A vector according to any preceding claim wherein the termination sequence corresponds to the terminator region located between nt 17265 and nt 17304 of the φ29 genome.

5. A vector according to any preceding claim further containing an heterologous promoter.

6. A vector according to claim 5 wherein the promoter is derived from φ29, or is equivalent thereto, or is a functional mutant form of either.

7. A vector according to any preceding claim which contains a promoter and has an expression sequence located between the promoter and terminator.

8. A vector according to any preceding claim which contains a promoter and has at least one restriction site between the promoter and terminator.

9. The plasmids pSHT3 and pSHT5.

10. A Streptomyces host cell containing a vector or plasmid according to any preceding claim.

11. A host cell according to claim 10 which is S. lividans.

12. An expression system for a protein using a host as defined in claim 10 or 11.

A a b c d e  B f g G A C T  C h i j  D k l G A C T

A-E
F
G
H
I
J
K

~690
~570
~470

L

78

M

N — —78

A-E
F
G
H
I
J
K

~690
~570
~470

L

78

M

N — —78

78  A3  A2b  A2c

470  570  690

A2a

E

A2a
690  570  470  pSH3
(7.3kb)

pSH5
(7.3 kb)

A2c  A3
A2b  78

neo  ┼ter┘

tsr

pIJ486
(6.2kb)

FIG.1.

FIG.2.

FIG.3.

XbaI    BamHI
GACTCTAGAGGATCCCCGGGTACCGAGCTCGAA

FIG.4.